# EUROPEAN PATENT APPLICATION

(11) **EP 4 491 629 A1**
(43) Date of publication of application: **15.01.2025**
(21) Application number: 23306218.1
(22) Date of filing: 13.07.2023
(51) Int. Cl.: C07K 14/47

(54) **EXPRESSION SYSTEM INCLUDING AN ENGINEERED LECTIN PROPERTY PURIFICATION TAG BASED ON GALECTIN-1**

(71) Applicant: Université de Lorraine, 54052 Nancy Cedex (FR); Centre National de la Recherche Scientifique, 75016 Paris (FR)
(72) Inventor: KRIZNIK, Alexandre, 54600 Villers-lès-Nancy (FR); REBOUL, Pascal, 54550 Sexey-aux-Forges (FR); CHARRON, Christophe, 54110 Flainval (FR)
(74) Representative: Novagraaf Technologies

(57) **Abstract**

The present invention concerns a new expression system including an engineered lectin property purification tag derived from galectin-1, in particular *Cricetulus griseus* galectin-1 .

## Description

### Technical field of the invention

The present invention concerns a new expression system including an engineered lectin property purification tag derived from galectin-1, in particular from *Cricetulus griseus* galectin-1.

The present invention finds its applications mainly in public and private research laboratories, as well as in the pharmaceutical industry having the need to produce recombinant proteins for the purpose of fundamental studies or of therapeutic interest.

In the description below, references in square brackets ([]) refer to the list of references at the end of the text.

### State of the art

For several years, the application needs for recombinant proteins have been growing in many economic and research fields, ranging, for example, from the pharmaceutical, chemical and agronomic industries to university laboratories.

A key step for the use of recombinant proteins is their purification from ectopic overexpression systems. For decades, bacterial strains have been optimised to improve the yields, purity, folding and/or safety of proteins encoded on plasmid expression vectors, which are themselves optimised to best perform their task. To facilitate operations, purification tags that can be fused to the protein of interest have been developed. Some tags, such as the SUMO (Small Ubiquitinlike MOdifier) tag, can improve the solubility of the protein construct, but many, including 6xHis (composed of at least six histidines), GST (glutathione S-transferase), MBP (Maltose-Binding Protein), TAP (Tandem Affinity Purification), FLAG and STREP (STREPtavidin), benefit from their ability to bind specifically to agarose or Sepharose resin beads coated with a specific ligand. The affinity for the ligand allows the protein of interest to be retained on the beads, while the proteins in the cell expression system are removed in the washing step. Competition with an eluent molecule, or direct cleavage by a protease specifically recognising a target region embedded in the protein construct, releases the protein of interest from the beads. Thus, the more specific the affinity chromatography step, the purer the eluted fractions.

Other methods of expression of proteins of interest allowing easy and efficient purification by affinity chromatography on low-cost resins, based on the use of lectins as tags, have been developed. The advantage of lectins is that they bind specifically and reversibly to certain polysaccharides. Indeed, there are several classes of lectins differing in their amino acid sequence, their three-dimensional structure but also in the nature of the binding site to sugars. Thus, methods for the expression and purification of proteins of interest have been developed from lectins such as the fungal lectin LSL [1] or the discoidin from an amoeba [2] on chromatography columns made of Sepharose 4B. However, in these methods, the use of an ungrafted resin leads to a risk of non-specific binding to the resin and thus contamination by bacterial proteins.

A method for the purification of proteins of interest using a resin grafted with mannose recognised by the *P. aeruginosa* lectin LecB is also known [3]. However, mannose is a sugar that is complex to synthesise and expensive.

The lectin capacities of galectins have been studied, in particular the properties of carbohydrate recognition domain (CRD) of human Galectin 3 [4, 5]. Recently a protein purification tag based on the carbohydrate recognition domain (CRD) of human galectin-3, called CRD_{SAT}, has been developed which allows for improved purification of proteins of interest by affinity chromatography. This protein tag has the advantage of being highly specific for its ligand, of facilitating the solubilisation of the protein to be purified and of working with a low cost system linked to the use of D-lactose [4]. More recently another protein purification tag, called CRD.VL, with retained lectin activity making it possible to select fusion proteins by affinity chromatography and improved stability at high temperature (up to about 92°C for instance), has been derived from CRD_{SAT}, which when fused to a thermostable protein of interest allows to improve the purification yield by simple heating.

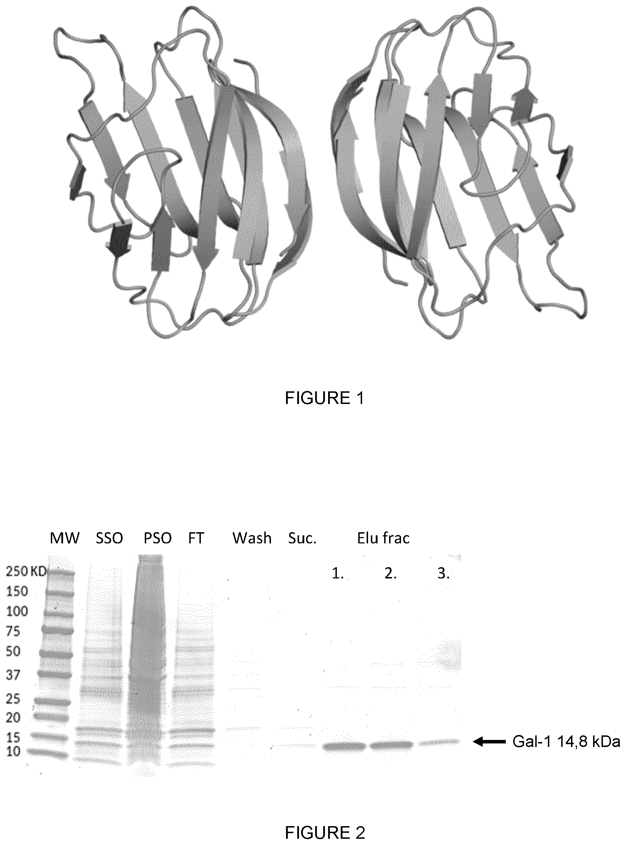

The human protein Galectin-1 (hGal1) has been described as suitable for use as a fusion tag for the capture and purification of a fusion protein [6]. However, it has an undesirable property. Indeed, hGal1 naturally self-assembles to generate a dimer, *i.e*. two assembled units (Fig. 1). This property interferes with the production and purification of recombinant proteins by generating unwanted complexes or generate aggregation phenomena that are deleterious and disabling for the user who wishes to obtain an isolated protein in perfect condition.

### Description of the invention

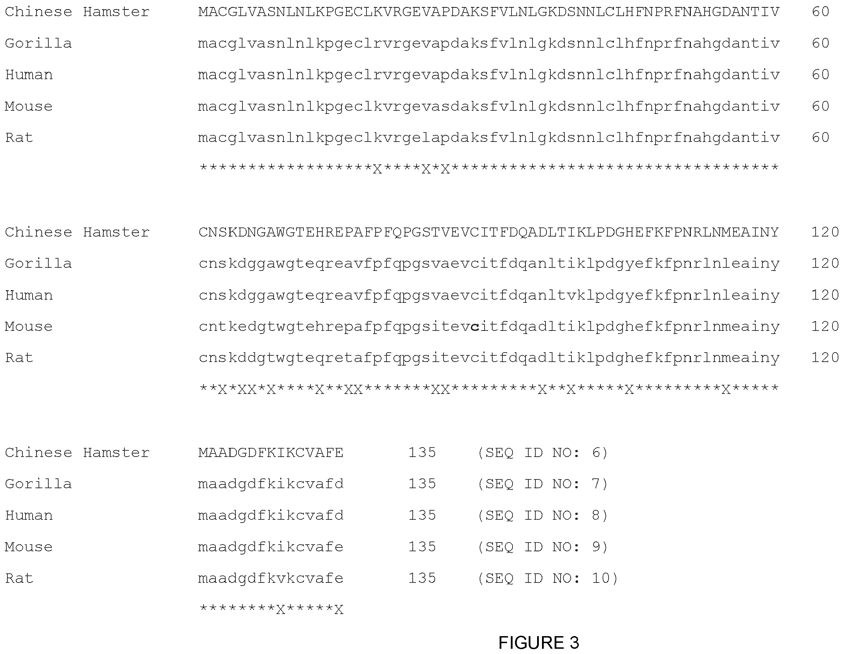

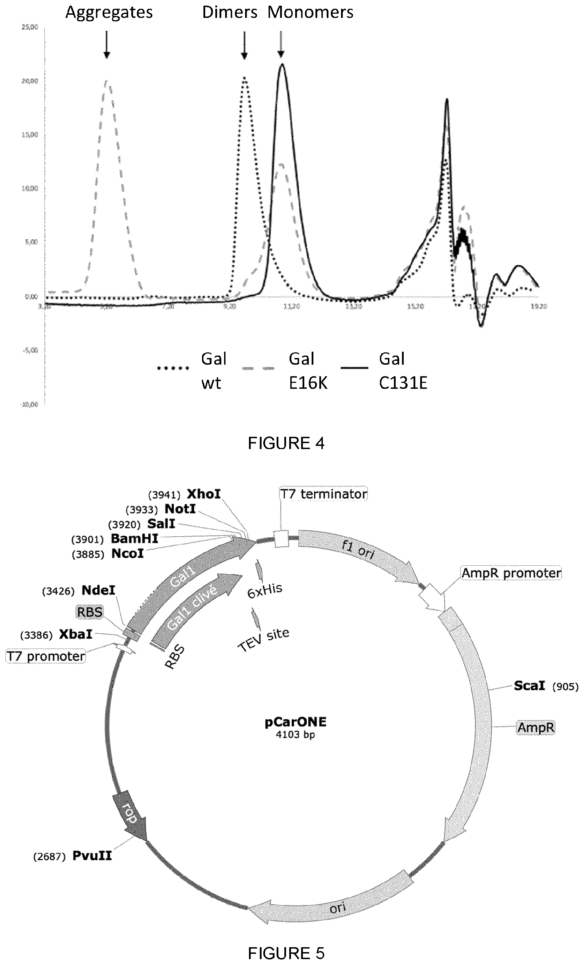

The Inventors have recently demonstrated, from a strain of Chinese hamster (*Cricetulus griseus*) cancer cells (CHO or ovarian cancer cells), the presence of a highly expressed and cleanly purified band on a polyacrylamide gel during a protein capture process by fixation on lactose-agarose or lactose-Sepharose (Fig. 2). This protein, identified as galectin-1 from this organism (sequence ID: XP_003510318.1; SEQ ID NO: 6), is produced in high quantities in a stable and soluble manner. This protein has 92% sequence identity with gorilla Gal-1, 91% sequence identity with human Gal-1, 94% sequence identity with mouse Gal-1, and 94% sequence identity with rat Gal-1 (Fig. 3). The nucleic acid sequence coding for this protein was inserted into a plasmid expression vector (pCarONE vector), to make use of its properties and its ability to bind a diholoside (lactose) in a very specific way. Unfortunately, the protein obtained was also unsatisfactory because it has the property of self-assembling, generating higher molecular weight complexes (dotted black line; Fig. 4), incompatible with further experimental processes.

Molecular engineering can often be used to modify the catalytic, binding or physicochemical properties of proteins. Different approaches are possible, some consisting of random mutagenesis of the peptide sequence, others based on a rationale guided by the three-dimensional structure of the protein. The inventors opted for this second approach to try to design a lectin property purification tag derived from galectin-1.

Therefore the Inventors have designed variants of *Cricetulus griseus* galectin-1 with retained lectin activity for expressing and purifying proteins of interest with the advantage of not modifying and/or impairing the activity of said proteins. Such variants were designed by making different mutations at the dimerisation interface in order to counteract this natural interaction and to obtain strongly expressed, monomeric, non-aggregating but also stable proteins that retained its lectin-like property. These monomeric variants of galectin-1, when fused to a protein of interest, allow the purification of said proteins with high specificity and high yield in a single affinity chromatography step using a Sepharose resin grafted with lactose molecules.

An object of the present invention is therefore a variant sequence of mammalian galectin-1 comprising or consisting of the following consensus sequence: 
MACGLVASNLNLKPGECLX19VRGEX24AX26DAKSFVLNLGKDSNNLCLHFNPR
FNAHGDANTIVCNX63KX65X66GX68WGTEX73REX76X77FPFQPGSX85X86EVCIT
FDQAX96LTX99KLPDGX105EFKFPNRLNX115EAINYMAADGDFKX129KX131VAFX
135 (SEQ ID NO: 1),
 wherein: X¹⁹ is K or R; X²⁴ is V or L; X²⁶ is P or S; X⁶³ is S or T; X⁶⁵ is D or E; X⁶⁶ is N, G, or D; X⁶⁸ is A or T; X⁷³ is H or Q; X⁷⁶ is P, A, or T; X⁷⁷ is A or V; X⁸⁵ is T, V, or I; X⁸⁶ is V, A or T; X⁹⁶ is D or N; X⁹⁹ is I or V; X¹⁰⁵ is H or Y; X¹¹⁵ is M or L; X¹²⁹ is I or V; and X¹³¹ is a negatively charged amino acid, preferably is D or E, more preferably is E, X¹³⁵ is E or D.

According to a particular embodiment of the present invention, the variant sequence of the invention comprises or consists of the following sequence:

MACGLVASNLNLKPGECLKVRGEVAPDAKSFVLNLGKDSNNLCLHFNPRFNA
HGDANTIVCNSKDNGAWGTEHREPAFPFQPGSTVEVCITFDQADLTIKLPDGH
EFKFPNRLNMEAINYMAADGDFKIKEVAFE (SEQ ID NO: 2),

MACGLVASNLNLKPGECLKV
RGEVAPDAKSFVLNLGKDSNNLCLHFNPRFNAHGDANTIVCNSKDNGAWGTE
HREPAFPFQPGSTVEVCITFDQADLTIKLPDGHEFKFPNRLNMEAINYMAADG
DFKIKDVAFE (SEQ ID NO: 3).

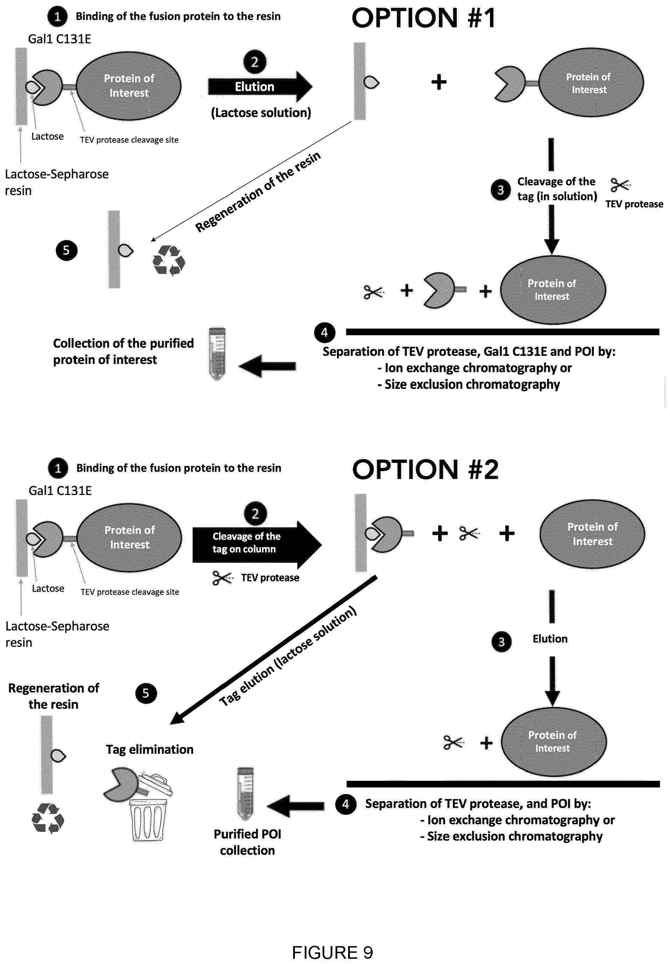

Another object of the present invention is a fusion protein comprising or consisting of a variant sequence of galectin-1 of the present invention fused with a protein of interest, with a sequence comprising a site for cleavage by protease inserted between the two (two options of cleavage are possible, see Fig. 9).

According to a particular embodiment of the present invention, the cleavage site is a site for cleavage by TEV protease.

Another object of the present invention is an expression vector comprising a nucleotide sequence encoding a fusion protein of the present invention.

According to a particular embodiment of the present invention, the expression vector comprises the following functionally linked elements:
a) a promoter placed 5' of the elements b), c) and d);
b) a nucleic sequence encoding the variant sequence of galectin-1 of the present invention;
c) a cloning site receiving a sequence encoding a protein of interest;
d) transcription termination signals;
and wherein a site for cleavage by protease is located between the sequence b) and the cloning site c).

"Promoter" as used herein means a cis-acting DNA sequence located 5' to the transcription initiation site of the polypeptide coding sequence to which a DNA sequence of a RNA polymerase can bind and initiate correct transcription, and optionally including activators.

According to a particular embodiment of the expression vector of the present invention, the sequence b) is placed near to the cloning site c), 3' of the promoter.

According to a particular embodiment of the expression vector of the present invention, the site for cleavage by protease is a site for cleavage by TEV protease.

According to a particular embodiment of the expression vector of the present invention, said vector is the pCarONE expression vector derived from the pET20b(+) vector and comprising the following elements:
an origin of replication at position 1630-2218;
a T7 promoter at position 3348-3366;
a ribosome binding site (RBS) at position 3398-3420;
the Gal1_{C131E} sequence at position 3428-3967;
a TEV recognition site sequence at position 3863-3883;
a multiple cloning site at position 3885-3941;
a T7 terminator at position 4031-4078;
an F1 origin at position 12-467; and
a *bla* ampicillin resistance gene at position 599-1459.

Another object of the present invention is a method for producing a purified protein of interest comprising (i) a prokaryotic heterologous production system, and (ii) purification of the protein of interest on lactose-agarose or Sepharose. Another object of the present invention is therefore a method for producing a purified protein of interest, said method comprising:
a) preparing an expression vector according to any one of claims 6 to 10;
b) transforming a host cell with said expression vector;
c) culturing said host cell transformed in this way under conditions enabling the expression of the fusion protein and promoting the dissolution thereof in host cells; and
d) purifying the molecule of interest.

According to a particular embodiment of the method of the present invention, the purification step d) is carried out by:
(i) binding the fusion protein, via the lectin domain of the variant sequence of galectin-1, to a chromatography support grafted with lactose molecules, preferably on a column of Sepharose or agarose resin grafted with lactose molecules;
(ii) cleavage by protease of the protein of interest;
(iii) elution of the protein of interest;
(iv) separation of the protease and the protein of interest.

According to a particular embodiment of the method of the present invention, the purification step d) is carried out by:
(i) binding the fusion protein, via the lectin domain of the galectin-1 or part of said domain, to a chromatography support grafted with lactose molecules, preferably on a column of Sepharose or agarose resin grafted with lactose molecules;
(ii) elution of the fusion protein;
(iii) cleavage by protease of the protein of interest in solution;
(iv) separation of the protease and the lectin domain or part of said domain from the protein of interest.

According to a particular embodiment of the method of the present invention, the separation step (iv) is carried out by ion exchange chromatography or size exclusion chromatography or hydrophobic interaction chromatography.

According to a particular embodiment of the method of the present invention, the elution step (iii) is carried out with a lactose solution.

### Brief description of the figures

Figure 1 shows the three-dimensional structure of the human Galectin-1 dimer obtained by solution NMR (Nesmelova et al., J. Mol. Biol., 2010) [7]
Figure 2 represents SDS-PAGE gel assessment of the production and purification of endogenous proteins from *Cricetulus griseus,* **MT:** Size marker, **SSO:** sonication supernatant before purification, **PSO:** sonication pellet, **Flow Through:** fraction not retained on lactose-Sepharose resin, **Wash:** wash with saline buffer, **Sucrose:** wash with buffer + sucrose, **Elution Fractions 1, 2 & 3:** elution fractions with Buffer + lactose. Band identification by mass spectrometry (MALDI-TOF): Galectin-1 OS from *Cricetulus griseus* GN=LGALS1 PE=1 SV=2; 14.8 kDa.
Figure 3 represents a peptide sequence alignment of mammalian galectin-1 (Gal-1) from Gorilla, Human, Mouse and Rat, *versus* Gal-1 from Chinese Hamster (Cys¹³¹ is conserved in all sequences).
Figure 4 represents the size exclusion column chromatography (Superdex 75 (Cytiva)) of purified Galectins. In dotted black, Gal1 wild-type; In dashed grey, Gal1 mutant E16K; and in solid black, Gal1 mutant C131E.
Figure 5 represents map of the plasmid vector pCarONE Gal-1 wt.
Figure 6 represents the main characteristics of the *pCarONE_{C131E} plasmid* (SEQ ID NOs: 4 and 11).
Figure 7 represents SDS-PAGE gel summary of the production and purification of the Gal1 C131E mutant from Cricetulus griseus, **MT:** size marker, **SSO:** soluble bacterial fraction = sonication supernatant before purification, **LS:** elution fraction with buffer + lactose, **TEV dialysis:** overnight dialysis in the presence of TEV protease, **After -80°C:** freezing of the sample at -80°C, **S75:** final stage of separation on a Superdex 75 size exclusion column.
Figure 8 represents SDS-PAGE gel summary of the production and purification of the fusion protein C131E mutant - protein RAGE **MT:** size marker, **SSO:** soluble bacterial fraction = sonication surpernatant before purification, **LS:** elution fraction with buffer + lactose.
Figure 9 represents the scheme of the lactose affinity purification method of the present invention.

### EXAMPLES

### EXAMPLE 1: PRODUCTION OF MONOMERIC LECTIN PROPERTY PURIFICATION TAGS DERIVED FROM CRICETULUS GRISEUS GALECTIN-1

### A- Design of different mutations at the dimerisation interface of Cricetulus griseus galectin-1

An observation of the three-dimensional solution NMR (PDB:2KM2) structure of the *Cricetulus griseus* galectine-1 was undertook to carry out mutations on positions at the dimerisation interface in order to counteract the natural dimerisation of the protein and to obtain strongly expressed, monomeric, non-aggregating but also stable proteins that retained its lectin-like property.

Taken advantage of these positions, two protein sequences were designed, called C131E mutant (SEQ ID NO: 2) and E16K mutant (SEQ ID NO: 5).

From the sequence of C131E mutant, and alignment of mammalian Gal-1 from gorilla, human, mouse and rat, a consensus sequence SEQ ID NO: 1 was derived.

### B- Production and purification of the C131E mutant and E16K mutant

The pCarONE plasmid is derived from Novagen's pET20b(+) and comprises the following elements (Fig. 5):

| Elements | Position |
|---|---|
| Origin of replication (ori) | 1630-2218 |
| T7 promoter | 3348-3366 |
| Ribosome Binding site (RBS) | 3398-3420 |
| mutant Gal-1 sequence | 3428-3967 |
| TEV recognition site sequence | 3863-3883 |
| Multiple cloning site (MCS) | 3885-3941 |
| T7 terminator | 4031-4078 |
| F1 origin | 12-467 |
| bla gene (ampicillin resistance) | 599-1459 |

The pCarONE plasmid allows the production of a variant sequence of galectin-1 consisting of, in order: a mutant Gal-1 sequence, a spacer arm allowing flexibility, a TEV protease cleavage site.

The *E*. *coli* strain used for its expression must be of the (DE3) type, *i.e*. having the T7 RNA polymerase gene integrated into its genome.

### 1. Construction of the pCarONE expression vector

The *E.coli* codon optimized nucleic acid sequence coding for *Cricetulus griseus* galectin-1 was inserted into the pET20b(+) vector, generating the pCarONE vector. For creation of pCarONEc_{131E} and pCarONE_{E16K} variants, the nucleic acid sequence coding for the C131E mutant or the E16K mutant of *Cricetulus griseus* galectin-1 were obtained by PCR-mediated directed mutagenesis.

### 2. Expression of the C131E mutant and E16K mutant

A BL21(DE3) *Escherichia coli* strain was transformed with pCarONEc_{131E} or pCarONE_{E16K} and cultured into a LB medium, then an induction of the protein Gal1 C131E or Gal1 E16K expression was allowed by adding the inductor IPTG 1 mM during the exponential growth phase of the organism. After an overnight culture at 20°C, the culture were harvested by centrifugation and the pellet was stored at -20°C.

The pCarONEc_{131E} and pCarONE_{E16K} allow the production of a variant sequence of galectin-1 consisting of, in order: the sequence of C131E mutant or E16K mutant, respectively, a spacer arm allowing flexibility, a TEV protease cleavage site.

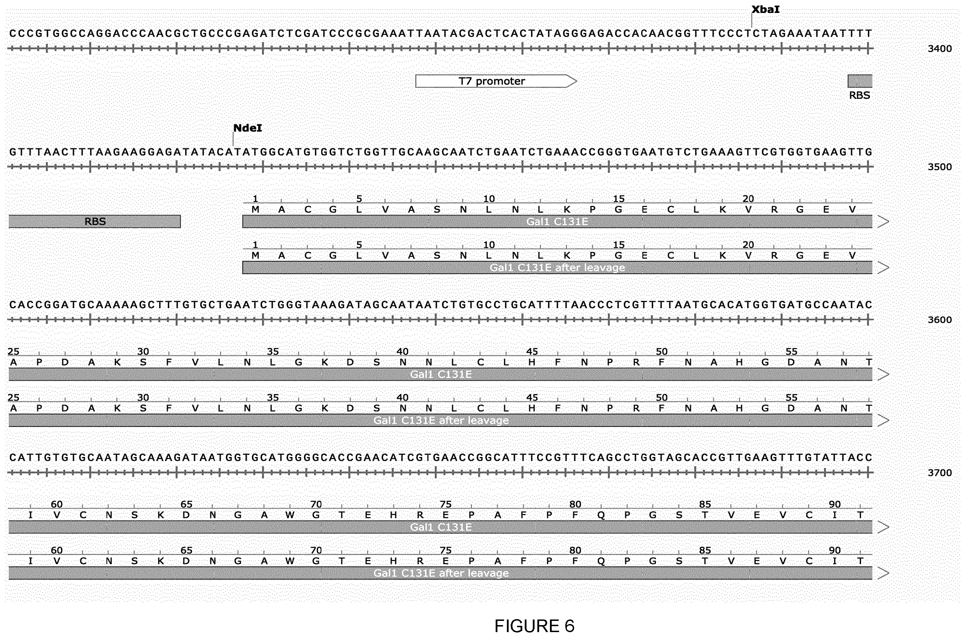

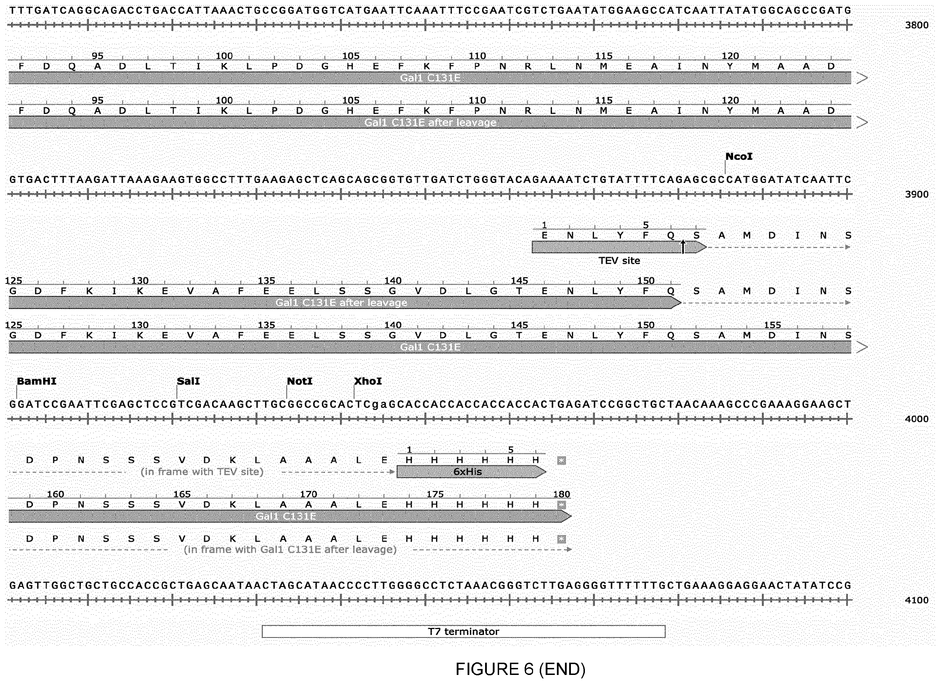

The main characteristics of pCarONEc_{131E} plasmid are shown in Fig. 6 (SEQ ID NOs : 4 and 11).

### 3. Purification of the C131E mutant and E16K mutant (TEV protease cleavage)

After centrifugation (4000 g for 20 min) of culture obtained as described above, each bacterial pellet was suspended in 10 mL of lysis buffer. The slurry was sonicated at 4°C for 2 cycles of 1 min at 80 watts then, centrifuged at 30, 000 g for 30 min. The filtrate was poured onto a pre-equilibrated with equilibration buffer, 1 ml lactose-Sepharose column (Galab Laboratories, Hamburg, Germany) previously equilibrated. The column was washed with 6 column volumes (CV) of washing buffer and elution was obtained with 1 CV of elution buffer.
Lysis buffer: NaPi 20 mM pH 7.5, NaCl 300 mM, EDTA 2 mM containing 4 mM MgSO4 and 5 U/mL Denarase^{®} (C-Lecta, Leipzig, Germany).
Equilibration buffer: NaPi 20 mM, pH 7.5 containing NaCl 300 mM, EDTA 2 mM.
Washing buffer: NaPi 20 mM, pH 7.5, NaCl 300 mM.
Elution buffer: NaPi 20 mM, pH 7.5 containing NaCl 150 mM and lactose 100 mM.

### C- Structural analysis of C131E mutant and E16K mutant

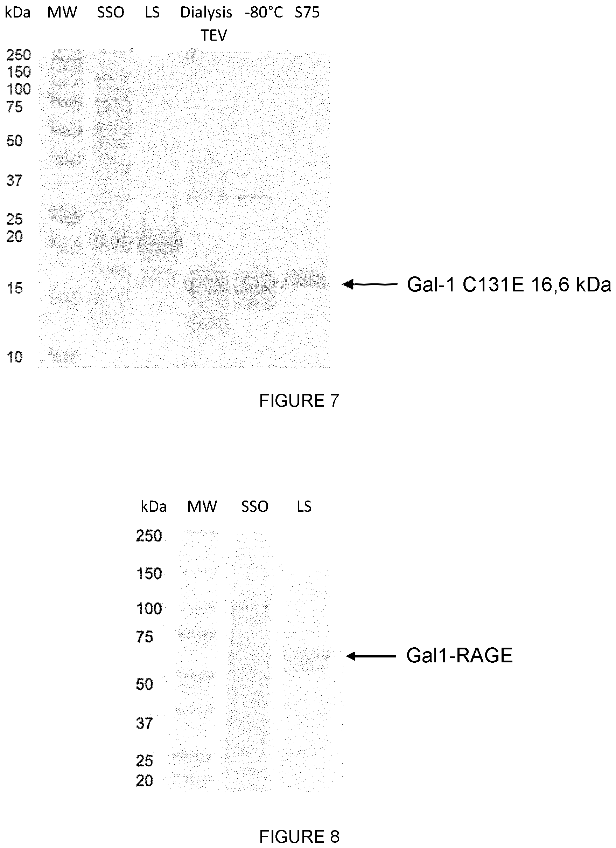

Two mutants were generated (E16K and C131E), produced and purified. The interaction was disrupted either by reversing the charge of the corresponding amino acid (E negative charge to K positive charge) or by adding a charge in place of an uncharged amino acid (C uncharged to E negatively charged). The presence of the lectin property of these mutants was validated by purifying them by affinity chromatography on lactose-Sepharose (*e.g*. as shown for mutant C131E, Fig. 7).

These mutants were then characterised by gel filtration or size exclusion chromatography to determine their oligomeric state (dashed grey: Gal1 mutant E16K; solid black: Gal1 mutant C131E; Fig. 4). The Gal1 chromatographic peak (doted black line; Fig. 4) appears first due to the larger size (2x16.6 kDa) of the dimer.

The E16K mutant (dashed grey line) is monomeric (later elution) but still contains a proportion of dimeric form, and many aggregates are formed and detected at the beginning of elution.

The C131E mutant on the other hand is totally monomeric and homogeneous (solid black line; Fig. 4). This mutation thus discovered and inserted in the Gal1 sequence allows to obtain only a form of the monomeric, stable, non-aggregating protein tag with high expression and retaining its lectin-like property.

### EXAMPLE 2 : USE OF C131E MUTANT TO PURIFY THE PROTEIN RAGE (Receptor of Advanced Glycation Ends)

### A- Construct of a fusion protein C131E mutant - protein RAGE (50,8 kDa)

### 1. Cloning into the pCarONEc_{131E} plasmid

A coding sequence for the extracellular domain of RAGE were produced in shuttle plasmids (pMAt, GeneArt Life Technologies). Sequence were subcloned into pCarONEc_{131E} using Ncol / Xhol sites. The generated vector (pCarONE_{C131E-RAGE} plasmid) is used to produce fused recombinant proteins with the Gal1 _{C131E} located at the N terminal position.

### 2. Expression of the fusion protein « Gal1 C131E mutant - protein RAGE »

A BL21(DE3) *Escherichia coli* strain was transformed with the pCarONE_{C131E-RAGE} plasmid and cultured into a LB medium, then an induction of the fusion protein Gal1 C131E-RAGE expression was allowed by adding the inductor IPTG 0.5 mM during the exponential growth phase of the organism. After an overnight culture at 20°C, the culture were harvested by centrifugation and the pellet was stored at -20°C.

pCarONE_{C131E-RAGE} allows the production of a fusion protein consisting of, in order: the C131E mutant of *Cricetulus griseus* galectin-1 (SEQ ID NO : 2), a spacer arm allowing flexibility (ELSSGVDLGT, SEQ ID NO : 13), a TEV protease cleavage site (ENLYFQ_{▲}S, SEQ NO : 14), and the protein of interest (protein RAGE).

### 3. Purification of the fusion protein « C131E mutant - protein RAGE »

The fusion protein C131E mutant - protein RAGE was purified on a lactose-Sepharose resin as described above (B.3.) (Fig. 8).

### B- Optional TEV protease cleavage of the fusion protein

Fusion proteins can be cleaved overnight at 4°C using TEV S219V mutant protease at a 1:100 w/w ratio. Fused proteins of interest (POIs) were separated from Gal1_{C131E} and TEV protease with an appropriate second chromatographic step (Fig. 9).

### List of references

- 1.: International Application WO 2009121994
- 2.: International Application WO 9966053
- 3.: Tielker et al., Biotechniques, 41(3): 327-332, 2006
- 4.: International Application WO 2017/194888
- 5.: Kriznik et al., Biotechnol. J., 2019
- 6.: Pasek et al., BBRC, 2010
- 7.: Nesmelova et al., J. Mol. Biol., 2010

## Claims

1. Variant sequence of galectin-1 comprising the following consensus sequence
MACGLVASNLNLKPGECL**X**¹⁹VRGE**X**²⁴A**X**²⁶DAKSFVLNLGKDSNNLCLHF NPRFNAHGDANTIVCN**X**⁶³K**X**⁶⁵**X**⁶⁶G**X**⁶⁸WGTE**X**⁷³RE**X**⁷⁶**X**⁷⁷FPFQPGS**X**⁸⁵**X**⁸⁶EV CITFDQA**X**⁹⁶LT**X**⁹⁹KLPDG**X**¹⁰⁵EFKFPNRLN**X**¹¹⁵EAINYMAADGDFK**X**¹²⁹K**X**¹³¹V AF**X**¹³⁵ (SEQ ID NO: 1), wherein: X¹⁹ is K or R; X²⁴ is V or L; X²⁶ is P or S; X⁶³ is S or T; X⁶⁵ is D or E; X⁶⁶ is N, G, or D; X⁶⁸ is A or T; X⁷³ is H or Q; X⁷⁶ is P, A, or T; X⁷⁷ is A or V; X⁸⁵ is T, V, or I; X⁸⁶ is V, A or T; X⁹⁶ is D or N; X⁹⁹ is I or V; X¹⁰⁵ is H or Y; X¹¹⁵ is M or L; X¹²⁹ is I or V; X¹³¹
is a negatively charged amino acid; and X¹³⁵ is E or D.

2. Variant sequence of galectin-1, wherein X¹³¹ is a glutamic acid (E) or an aspartic acid (D), preferably a glutamic acid (E).

3. Variant sequence of galectine-1 according to claim 1 or 2, wherein the variant sequence has the sequence 
MACGLVASNLNLKPGECLKVRGEVAPDAKSF
VLNLGKDSNNLCLHFNPRFNAHGDANTIVCNSKDNGAWGTEHREPAFPFQP
GSTVEVCITFDQADLTIKLPDGHEFKFPNRLNMEAINYMAADGDFKIKEVAFE
(SEQ ID NO: 2)
 or

MACGLVASNLNLKPGECLKVRGEVAPDAKSFVLNLGKDSNNLCLHFNPRFN
AHGDANTIVCNSKDNGAWGTEHREPAFPFQPGSTVEVCITFDQADLTIKLPDG
HEFKFPNRLNMEAINYMAADGDFKIKDVAFE (SEQ ID NO: 3).

4. Fusion protein comprising a variant sequence of galectin-1 as defined in any one of claims 1 to 3 fused with a protein of interest, with a sequence comprising a site for cleavage by protease inserted between the two.

5. Fusion protein according to claim 4, wherein the cleavage site is a site for cleavage by TEV protease.

6. Expression vector comprising a nucleotide sequence encoding a fusion protein as defined in claim 4 or 5.

7. Expression vector according to claim 6, said vector comprising the following functionally linked elements:
a) a promoter placed 5' of the elements b), c) and d);
b) a nucleic sequence encoding the variant sequence of galectin-1 as defined in any one of claims 1 to 3;
c) a cloning site receiving a sequence encoding a protein of interest;
d) transcription termination signals;
and wherein a site for cleavage by protease is located between the sequence b) and the cloning site c).

8. Expression vector according to claim 7, wherein the sequence b) is placed near to the cloning site c), 3' of the promoter.

9. Expression vector according to claim 7, wherein the site for cleavage by protease is a site for cleavage by TEV protease.

10. Expression vector according to any one of claims 6 to 9, wherein said vector is the pCarONE expression vector derived from the pET20b(+) vector and comprising the following elements:
an origin of replication at position 1630-2218;
a T7 promoter at position 3348-3366;
a ribosome binding site (RBS) at position 3398-3420;
the Gal1 _{C131E} sequence at position 3428-3967;
a TEV recognition site sequence at position 3863-3883;
a multiple cloning site at position 3885-3941;
a T7 terminator at position 4031-4078;
an F1 origin at position 12-467; and
a *bla* ampicillin resistance gene at position 599-1459.

11. Method for producing a purified protein of interest, said method comprising:
a) preparing an expression vector according to any one of claims 6 to 10;
b) transforming a host cell with said expression vector;
c) culturing said host cell transformed in this way under conditions enabling the expression of the fusion protein and promoting the dissolution thereof in host cells; and
d) purifying the molecule of interest.

12. Method according to claim 11, wherein the purification step d) is carried out by:
(i) binding the fusion protein, via the lectin domain of the variant sequence of galectin-1, to a chromatography support grafted with lactose molecules, preferably on a column of Sepharose or agarose resin grafted with lactose molecules;
(ii) cleavage by protease of the protein of interest;
(iii) elution of the protein of interest;
(iv) separation of the protease and the protein of interest.

13. Method according to claim 11, wherein the purification step d) is carried out by:
(i) binding the fusion protein, via the lectin domain of the galectin-1 or part of said domain, to a chromatography support grafted with lactose molecules, preferably on a column of Sepharose or agarose resin grafted with lactose molecules;
(ii) elution of the fusion protein;
(iii) cleavage by protease of the protein of interest in solution;
(iv) separation of the protease and the lectin domain or part of said domain from the protein of interest.

14. Method according to either one of claims 12 or 13, in which the separation step (iv) is carried out by ion exchange chromatography or size exclusion chromatography or hydrophobic interaction chromatography.

15. Method according to any one of claims 12 or 13, in which the elution step (iii) is carried out with a lactose solution.
